# EUROPÄISCHE PATENTSCHRIFT

(11) **EP 2 219 708 B1**
(45) Veröffentlichungstag und Bekanntmachung des Hinweises auf die Patenterteilung: **22.02.2017**
(21) Anmeldenummer: 08859950.1
(22) Anmeldetag: 07.11.2008
(51) Int. Cl.: F16D 1/10, A61M 5/145

(54) **VORRICHTUNG ZUM LÖSBAREN KUPPELN EINER ANTRIEBSSPINDEL MIT DER DRÜCKVORRICHTUNG EINER SPRITZENPUMPE**
DEVICE FOR THE DETACHABLE COUPLING OF A DRIVING SPINDLE TO THE PRESSING DEVICE OF A SYRINGE PUMP
DISPOSITIF POUR LE COUPLAGE RÉVERSIBLE D'UNE BROCHE D'ENTRAÎNEMENT AU DISPOSITIF DE POUSSÉE D'UN POUSSE-SERINGUE

(30) Priorität: 11.12.2007 DE 102007059803
(43) Veröffentlichungstag der Anmeldung: 25.08.2010
(73) Patentinhaber: Fresenius Medical Care Deutschland GmbH, 61352 Bad Homburg (DE)
(72) Erfinder: EBERT, Manuel, 4500 Solothurn (CH)
(74) Vertreter: Herrmann, Uwe
(86) Internationale Anmeldenummer: PCT/DE2008/001824
(87) Internationale Veröffentlichungsnummer: WO 2009/074125

(56) Entgegenhaltungen:
- WO-A-02/083209
- WO-A1-2006/015648
- DE-A1- 10 243 279
- US-A- 2 734 504
- US-A- 4 273 122
- US-A- 4 529 401
- US-A- 4 767 277
- US-A- 5 971 963
- US-B1- 6 381 933
- US-B1- 6 461 111

## Beschreibung

Die Erfindung betrifft eine Spritzenpumpe mit einer Vorrichtung zum lösbaren Kuppeln einer Antriebsspindel der Spritzenpumpe mit einer Drückvorrichtung der Spritzenpumpe, wobei Mittel zum formschlüssigen Kuppeln der Antriebsspindel mit der Drückvorrichtung vorgesehen sind.

Bei den bekannten Spritzenpumpen wird eine (eventuell vorbefüllte) Spritze in die Spritzenpumpe so eingelegt, daß der Spritzenzylinder vom Gehäuse der Spritzenpumpe gehalten wird. Der Spritzenkolben, der zur Abgabe des Spritzeninhaltes in den Zylinder hineingedrückt wird, wird von einem Schieber betätigt. Um den Schieber relativ zum Gehäuse der Spritzenpumpe zu bewegen, ist ein Spindelantrieb vorgesehen, der die Rotationsbewegung des Antriebs in eine Translationsbewegung umsetzt.

Um beim Einlegen verschiedener Spritzengrößen oder unterschiedlich weit aufgezogener Spritzen den Schieber schnell bis zum Anschlag des Spritzenzylinders unter Umgehung des Antriebs bewegen zu können, wird der Antrieb von der Drückvorrichtung entkoppelt.

Die DE 38 38 465 A1 beschreibt eine Spritzenpumpe, bei der ein Kupplungselement vorgesehen ist, um ein Ein- und Ausrasten der Antriebselemente zu vermeiden. Das Kupplungselement, das aus zwei Zahnrädern oder aus einer Mutter bestehen kann, ist permanent mit der Antriebsspindel im Eingriff und wird über Bremselemente blockiert bzw. freigegeben. Die Bremselemente werden gegen die Wirkung einer Feder von einem Betätigungsknopf über ein Bremsrohr betätigt. Die Zahnräder, die mit der Antriebsspindel zusammenwirken, werden also durch die Bremselemente im Normalzustand blockiert. Über den Betätigungsknopf können die Bremselemente gelöst werden, um die Zahnräder freizugeben. Wenn der Schieber in Position ist, wird der Betätigungsknopf losgelassen und die Feder drückt das Bremsrohr wieder in die Ausgangsstellung, in der die Bremselemente die Zahnräder blockieren. Nachteilig ist bei dieser Vorrichtung, daß beim Kuppeln immer eine Kraft direkt auf die Gewinde der Antriebsspindel und der Antriebseinheit (Mutter bzw. Zahnräder) einwirkt. Durch die hohe Flankenpressung und aufgrund des zwangsläufig vorhandenen Winkelspiels zwischen den Flanken ist der Verschleiß groß, was auf die Dauer die Präzision der Pumpe beeinträchtigt. Zudem unterliegen die Bremselemente, die über Reibschluß die Zahnräder blockieren, einer relativ großen Abnutzung.

Aus der US 4,465,475 A ist eine kraftschlüssige Kupplung bekannt. Hier wird durch Betätigen eines Knopfes die Antriebsspindel in eine Richtung freigegeben. Ein Ring, der als Käfig für zwei Nadeln dient, wird durch die Knopfbetätigung gedreht und die Nadeln können entweder frei rotieren oder werden gegen die Antriebsspindel gepreßt und blockieren deren Rotationsbewegung. Dieser Aufbau ist jedoch kompliziert und ermöglicht zudem (durch Drehen eines Stellrades) eine Bewegung in Arbeitsrichtung der Drückvorrichtung, auch wenn der Knopf nicht gedrückt ist. Dies könnte zur unbeabsichtigten Abgabe des zu infundierenden Mittels führen, was nicht erwünscht ist.

Die WO 02/083209 A1, aus der der Oberbegriff gebildet wurde, beschreibt ein Antriebssystem für eine Infusionspumpe, die eine Vorrichtung zum lösbaren Kuppeln einer Antriebsspindel mit der Drückvorrichtung einer Spritzenpumpe aufweist, wobei Mittel zum formschlüssigen Kuppeln der Antriebsspindel mit der Drückvorrichtung vorgesehen sind.

Aus der US 5,971,963 A ist eine tragbare automatische Spritzenvorrichtung bekannt, die einen rotierenden Schaft zum Übertragen eines Vortriebs auf einen Kolben der automatischen Spritzenvorrichtung aufweist. Hierbei ist ein Kopplungsmittel zwischen dem Schaft und Kraftübertragungsmitteln vorgesehen.

Die US 4,273,122 A beschreibt eine Vorrichtung und ein Verfahren zum Injizieren von Flüssigkeiten mit einer definierten Durchflußrate in Patienten, wobei ein Kolben die Flüssigkeiten aus einer Ampulle in den Patienten schiebt.

Aus der US 2,734,504 A ist ebenfalls eine Injektionsvorrichtung bekannt, wobei hier der Vortrieb über ein Schraubgewinde erfolgt.

Die US 4,529,401 A beschreibt eine tragbare Infusionspumpe zum kontrollierten Verabreichen einer vorbestimmten Menge eines Arzneimittels an einen Patienten.

Aufgabe der Erfindung ist es somit, eine Vorrichtung gemäß dem Oberbegriff zu schaffen, die auch über lange Zeiträume eine große Genauigkeit der Spritzenpumpe ermöglicht, auch wenn hohe Drehmomente übertragen werden.

Diese Aufgabe wird erfindungsgemäß dadurch gelöst, daß als Mittel zum Kuppeln der Antriebsspindel mit der Drückvorrichtung ein erstes Kupplungselement mit einer Ausnehmung und ein zweites Kupplungselement mit einem entsprechend geformten Vorsprung zum Eingriff in die Ausnehmung vorgesehen sind und die Ausnehmung als Innensechsrund und der Vorsprung als Außensechsrund ausgebildet sind und daß die Mittel zum formschlüssigen Kuppeln der Antriebsspindel mit der Drückvorrichtung als zulaufende Kupplungselemente ausgebildet sind.

Dies bedeutet, daß im Eintrittsbereich der Kupplungselemente kein oder lediglich ein geringfügiger Formschluß besteht und erst mit zunehmender Eintrittstiefe, also fortschreitendem Kupplungsvorgang der vollständige Formschluß erreicht wird. Ideal ist in diesem Zusammenhang ein linearer Anstieg des Formschlusses vom Eintrittsbereich bis hin zum maximalen Formschluß, der nach etwa einem Drittel der Tiefe der Ausnehmung erreicht sein sollte, um das übertragbare Drehmoment nicht zu sehr zu reduzieren.

Beispielsweise bei einer Ausbildung des ersten und des zweiten Kupplungselementes als Innen- bzw. Außensechsrund ist der Innensechsrund im Bereich der Öffnung der Ausnehmung noch nicht oder nur in geringem Maße ausgebildet und nimmt erst mit zunehmender Tiefe (etwa bei einem Drittel der Tiefe der Ausnehmung) eine Form an, die im wesentlichen dem Außensechsrund (zuzüglich der erforderlichen Toleranz) entspricht. Entsprechendes kann auch für den Außensechsrund gelten. Die das Drehmoment übertragende Kante ist also im Eintrittsbereich der Ausnehmung bzw. des Vorsprungs weniger ausgeprägt und/oder abgerundet, was den Eingriff des Vorsprungs in die Ausnehmung erleichtert. Hierdurch wird erreicht, daß auch bei nicht exaktem Übereinanderliegen von Vorsprung und Ausnehmung beim Kupplungsvorgang ein sanftes Einkuppeln erfolgt.

Einerseits liegen also ein erstes Kupplungselement mit einer Ausnehmung (das als "Kupplungsglocke" bezeichnet werden kann), und ein zweites Kupplungselement mit einem Vorsprung zum Eingriff in die Ausnehmung des ersten Kupplungselementes, vor. Dieses zweite Kupplungselement kann als "Kupplungsmitnehmer" bezeichnet werden. Im gekuppelten Zustand ist der Vorsprung in Eingriff in der Ausnehmung, im ungekuppelten Zustand ist er nicht in Eingriff.

Sechskante (hier: eine Ausnehmung bzw. ein Vorsprung, die jeweils die Form eines regelmäßigen Sechsecks aufweisen) sind leicht herstellbar und ermöglichen gleichzeitig die Übertragung hoher Drehmomente.

Die Ausgestaltung als Sechsrund (sechszackiger Stern mit abgerundeten Spitzen und Ecken, also einer Wellenform, wie sie von Torx®-Schrauben-Mitnahmeprofilen bekannt ist) ermöglicht eine optimale Kraftübertragung. Das Wellenprofil erleichtert zudem das Eingreifen des zweiten Kupplungselementes in das erste Kupplungselement.

Bei einer ersten Ausbildung der Erfindung ist das erste Kupplungselement an der Antriebsspindel und das zweite Kupplungselement an dem Antrieb ausgebildet.

Bei einer zweiten Ausbildung der Erfindung ist das erste Kupplungselement an dem Antrieb und das zweite Kupplungselement an der Antriebsspindel ausgebildet.

Nachfolgend wird ein Ausführungsbeispiel der Erfindung anhand von Zeichnungen erläutert.

Es zeigen
- Fig. 1a, 1b und 1c: Darstellungen des ersten Kupplungselementes in geschnittener Darstellung, in Draufsicht und in perspektivischer Ansicht,
- Fig. 2a, 2b, und 2c: Darstellungen des zweiten Kupplungselementes in geschnittener Darstellung, in Draufsicht und in perspektivischer Ansicht,
- Fig. 3: eine geschnittene Darstellung einer Kupplungsvorrichtung gemäß der Erfindung.

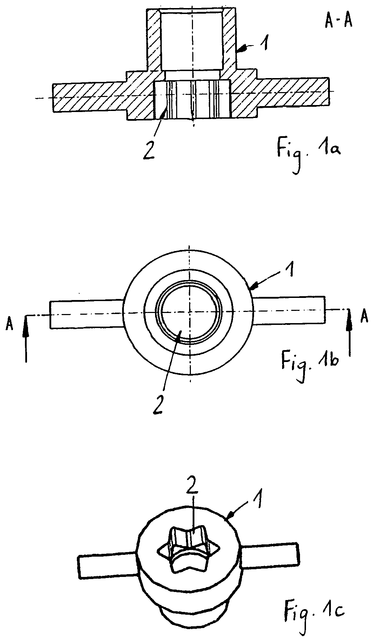

Wie aus den Fig. 1a, 1b und 1c ersichtlich, weist das erste Kupplungselement 1 eine polygonal geformte Ausnehmung 2 auf, die im vorliegenden Beispiel als Innensechsrund (gemäß ISO 10664-30, entsprechend einer Innentorx®-Ausbildung) ausgestaltet ist. Hierbei ist es vorteilhaft, daß der Innensechsrund zulaufend ausgebildet ist, d.h. daß mit zunehmender Tiefe der Ausnehmung 2 die Form des Innensechsrundes immer stärker ausgeprägt ist (vorzugsweise linear ansteigend), bis schließlich der Innensechsrund voll ausgebildet ist. Hierdurch wird erreicht, daß unabhängig von der Stellung des ersten Kupplungselementes 1 zu dem zweiten Kupplungselement 3 jeweils ein weiches Einkuppeln erfolgt. Die Tiefe, über die der Innensechsrund zuläuft, kann je nach den Anforderungen variieren, sollte jedoch ein Drittel des Innensechsrundes nicht überschreiten, damit das übertragbare Drehmoment nicht zu stark vermindert wird. Das erste Kupplungselement 1 weist eine Bohrung auf, durch die sich die Antriebsspindel erstreckt.

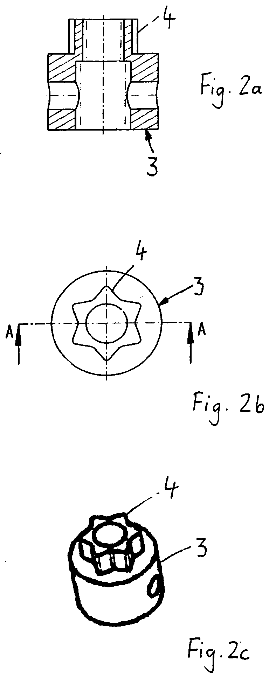

Das entsprechende zweite Kupplungselement 3 ist in den Fig. 2a, 2b und 2c dargestellt. Bei diesem ist beim vorliegenden Beispiel ein als Außensechsrund ausgebildeter Vorsprung 4 vorgesehen, dessen Außenmaße im wesentlichen (bis auf die Toleranz) den Innenmaßen des Innensechsrundes des ersten Kupplungselementes 1 entspricht und der somit zum formschlüssigen Eingriff in derselben ausgebildet ist. Das zweite Kupplungselement 3 weist ebenfalls eine zentrale Bohrung auf, um es auf die Antriebsspindel aufbringen zu können. Selbstverständlich kann auch der Vorsprung zulaufend ausgebildet sein bzw. sowohl die Ausnehmung als auch der Vorsprung.

Beim Kupplungsvorgang zwischen der Antriebsspindel und der Drückvorrichtung tritt der als Außensechsrund ausgebildete Vorsprung 4 des zweiten Kupplungselementes 3 in die Ausnehmung 2 des ersten Kupplungselementes 1 ein und es bildet sich mit zunehmender Eindringtiefe ein sicherer Formschluß zwischen dem Außensechsrund des zweiten Kupplungselementes 3 und dem Innensechsrund des ersten Kupplungselementes 1 aus.

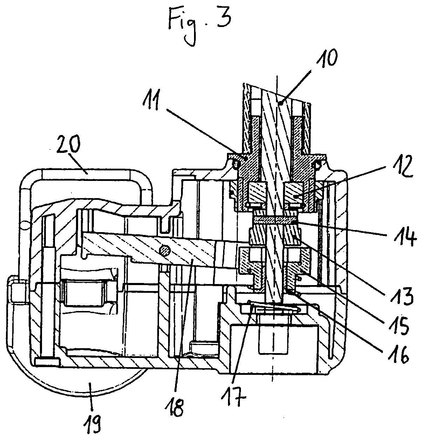

In Fig. 3 ist eine Kupplungsvorrichtung gemäß der Erfindung dargestellt. Das untere Ende einer Antriebsspindel 10 ist in einem Lagergehäuse 11 mittels eines Kugellagers 12 axial drehbar gelagert.

Ein Außensechsrund 13, der eine Durchgangsbohrung aufweist, ist auf das untere Ende der Antriebsspindel 10 aufgeschoben und mittels einer Verstiftung 14 an ihr befestigt.

Diesem Außensechsrund 13 gegenüberliegend ist ein Innensechsrund 15 mittels eines Gleitlagers 16 axial entlang der Antriebsspindel 10 verschiebbar angeordnet. Der Außensechsrund 13 ist zum Eingriff in den Innensechsrund 15 ausgebildet. Eine Feder 17 drückt im Normalzustand das Innensechsrund 15 in das Außensechsrund 13, so daß Innensechsrund 15 und Außensechsrund 13 kuppelnd in Eingriff sind.

Um die Antriebsspindel 10 (und somit den Antrieb) von der Drückvorrichtung zu entkoppeln, kann das Innensechsrund 15 über einen Schwenkhebel 18 gegen die Wirkung der Feder 17 aus dem Eingriff mit dem Außensechsrund 13 gelöst und damit ein schnelles axiales Verschieben der Drückvorrichtung für die Spritze (nicht dargestellt) ermöglicht werden. Der Schwenkhebel 18 wird durch das Zusammendrücken der Hebel 19 (gegen die Wirkung einer weiteren Feder), an deren Enden 20 der Spritzenkolben gehaltert ist, betätigt.

## Patentansprüche

1. Spritzenpumpe mit einer Vorrichtung zum lösbaren Kuppeln einer Antriebsspindel (10) der Spritzenpumpe mit einer Drückvorrichtung der Spritzenpumpe, wobei Mittel (2, 4) zum formschlüssigen Kuppeln der Antriebsspindel (10) mit der Drückvorrichtung vorgesehen sind, wobei als Mittel (2, 4) zum Kuppeln der Antriebsspindel (10) mit der Drückvorrichtung ein erstes Kupplungselement (1) mit einer Ausnehmung (2) und ein zweites Kupplungselement (3) mit einem entsprechend geformten Vorsprung (4) zum Eingriff in die Ausnehmung (2) vorgesehen sind und die Ausnehmung (2) als Innensechsrund und der Vorsprung (4) als Außensechsrund ausgebildet sind und wobei die Mittel (2, 4) zum formschlüssigen Kuppeln der Antriebsspindel (10) mit der Drückvorrichtung als zulaufende Kupplungselemente (2, 4) ausgebildet sind.

2. Spritzenpumpe gemäß Anspruch 1, **dadurch gekennzeichnet, daß** das erste Kupplungselement (1) an der Antriebsspindel (10) und das zweite Kupplungselement (3) an der Drückvorrichtung ausgebildet ist.

3. Spritzenpumpe gemäß Anspruch 1, **dadurch gekennzeichnet, daß** das erste Kupplungselement (1) an der Drückvorrichtung und das zweite Kupplungselement (3) an der Antriebsspindel (10) ausgebildet ist.

## Claims

1. A syringe pump having an apparatus for a releasable coupling of a drive spindle (10) of the syringe pump with an ejection apparatus of the syringe pump, wherein means (2, 4) are provided for the shape-matched coupling of the drive spindle (10) with the ejection apparatus, wherein
a first coupling element (1) having a recess (2) and a second coupling element (3) having a correspondingly shaped projection (4) for engagement into the recess (2) are provided as means (2, 4) for the coupling of the drive spindle (10) with the ejection apparatus and the recess (2) is configured as an internal star and the projection (4) is configured as an external star; and wherein
the means (2, 4) for the shape-matched coupling of the drive spindle (10) with the ejection apparatus are configured as tapering coupling elements (2, 4).

2. A syringe pump in accordance with claim 1, **characterized in that** the first coupling element (1) is formed at the drive spindle (10) and the second coupling element (3) is formed at the ejection apparatus.

3. A syringe pump in accordance with claim 1, **characterized in that** the first coupling element (1) is formed at the ejection apparatus and the second coupling element (3) is formed at the drive spindle (10).

## Revendications

1. Pousse-seringue comprenant un dispositif pour le couplage réversible d'une broche d'entraînement (10) du pousse-seringue au dispositif de poussée du pousse-seringue, dans lequel des moyens (2, 4) de couplage par complémentarité de formes de la broche d'entraînement (10) au dispositif de poussée sont prévus, dans lequel
un premier élément de couplage (1) doté d'un évidement (2) et un second élément de couplage (3) doté d'une saillie (4) formée de manière correspondante pour la mise en prise dans l'évidement (2) sont prévus en tant que moyens (2,4) de couplage de la broche d'entraînement (10) au dispositif de poussée et l'évidement (2) est réalisé sous la forme d'empreinte à six lobes internes et la saillie (4) sous la forme de tête à six lobes externes et dans lequel
les moyens (2, 4) de couplage par complémentarité de formes de la broche d'entraînement (10) au dispositif de poussée sont réalisés sous la forme d'éléments de couplage (2, 4) coniques.

2. Pousse-seringue selon la revendication 1, **caractérisé en ce que** le premier élément de couplage (1) est réalisé sur la broche d'entraînement (10) et le second élément de couplage (3) sur le dispositif de poussée.

3. Pousse-seringue selon la revendication 1, **caractérisé en ce que** le premier élément de couplage (1) est réalisé sur le dispositif de poussée et le second élément de couplage (3) sur la broche d'entraînement (10).
